# EUROPEAN PATENT SPECIFICATION

(11) **EP 2 016 917 B1**
(45) Date of publication and mention of the grant of the patent: **22.12.2010**
(21) Application number: 07014352.4
(22) Date of filing: 20.07.2007
(51) Int. Cl.: A61B 17/70

(54) **Implant for supporting spinous processes**
Implantat zum Stützen von Dornfortsätzen
Implant pour le soutien des apophyse épineuses

(43) Date of publication of application: 21.01.2009
(73) Proprietor: BioMed Ltd., Apia (WS)
(72) Inventor: Yeh, Chung-Chun, Taipei (TW)
(74) Representative: Lang, Christian

(56) References cited:
- EP-A- 1 138 268
- WO-A-2005/110258
- WO-A-2006/135889
- WO-A-2007/034516

## Description

### FIELD OF THE INVENTION

The invention relates to an implant for supporting spinous processes, and more particularly, to an implant for supporting spinous processes with two connectable and rotatable props that may be applied to neighboring spinous processes, and rotated to enlarge and support the intervertebral space between the neighboring vertebrae being treated.

### BACKGROUND OF THE INVENTION

Traditionally, afflictions related to the spine can be caused by external injuries like fractures, or diseases and atrophy of the spinal discs, which take up a large proportion. Diseases and atrophy of the spinal discs may reduce the intervertebral space, and results in the reduction of the foramina between the intervertebral joints. Subsequently, it leads to numbness or pain in an afflicted person due to compression of spinal nerve roots.

The currently available treatments to the aforesaid problem include implanting pedicle screws into the spine to stretch the intervertebral space, or implanting cages or spacers to fill the intervertebral space and enlarge the foramina. However, such treatments are surgically complicated and laborious to carry out, and still have issues related to efficiency and safety; thus an efficient, surgically simple and safe alternative is urgently required.

The key to solving the aforesaid problem is about enlarging the foramen and relieving the compressed spinal nerve roots, while also keeping the related surgery effective and simple. Therefore, a device has been proposed that can directly support the spinous processes of two neighboring vertebrae, which effectively enlarges the intervertebral space therebetween and solving the problem of compressed spinal nerve roots, thus treating numbness and pain in afflicted persons.

Related prior art is disclosed in WO 2006/135889 A2, WO 2007/034516 A1, WO 2005/110258 A1 and EP 1 138 268 A1.

### SUMMARY OF THE INVENTION

To solve the aforesaid problem, a plurality of props are required, which are rotatably connected and thus allowing an angle to be formed between the props; the props are paired in application with receiving ends at two ends for contacting with the spinous processes, and then rotated to increase the angle therebetween in order to make the props appear linear as a whole. As a result, the intervertebral space may be enlarged and supported, which solves the problem of reduced intervertebral space.

Therefore, a major objective of the invention is to propose an implant for supporting spinous processes comprising the aforesaid plurality of props.

An implant for supporting spinous processes constructed according to the present invention is defined in claim 1.

The fixing member is a cylindrical piece with a flat surface formed at two opposing sides thereof, the two flat surfaces of the fixing member separately contact the upper and lower hooks, when the fixing member is rotated to the second position; cylindrical surfaces separated by the two flat surfaces of the fixing member contact the upper and lower hooks, when the fixing member is rotated to the first position.

Alternatively not part of the invention, but important for its understanding, the connecting end of the second prop further comprises an axis having a fastening clip, and the connecting end of the first prop further comprises an axial trough having a fastening groove formed on an inner surface thereof; the axis being rotatably fitted into the axial trough while the fastening clip being retracted by pressing from the inner surface of the axial trough, when the two props are rotated to from an angle in relation to each other; the fastening clip springing out and clipping into the fastening groove, thus preventing the props from being rotated in relation to each other further, when the two props are rotated to become linear as a whole.

In regard to the relative positions defined in the invention like the upper side, the lower side, and the lateral sides; the positions are defined under the circumstance in which the position of the patient's head being the upper side, while the direction of the patient's feet being the lower side; the sides that are not the upper or the lower sides being the lateral sides.

### BRIEF DESCRIPTION OF DRAWINGS

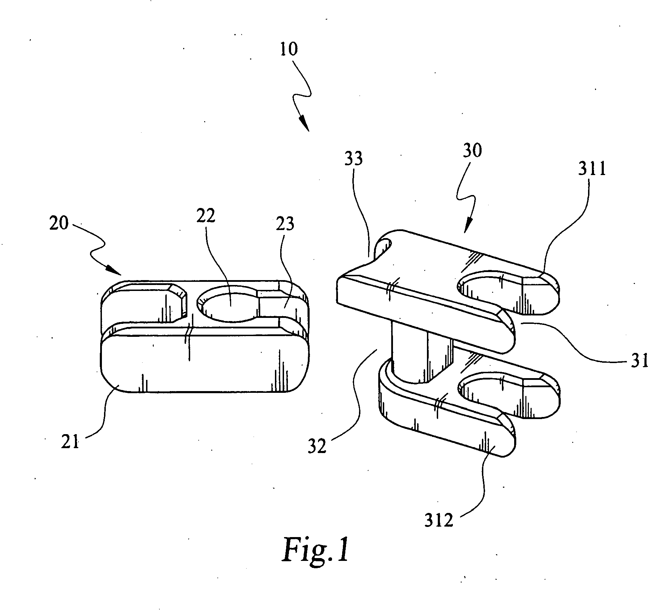

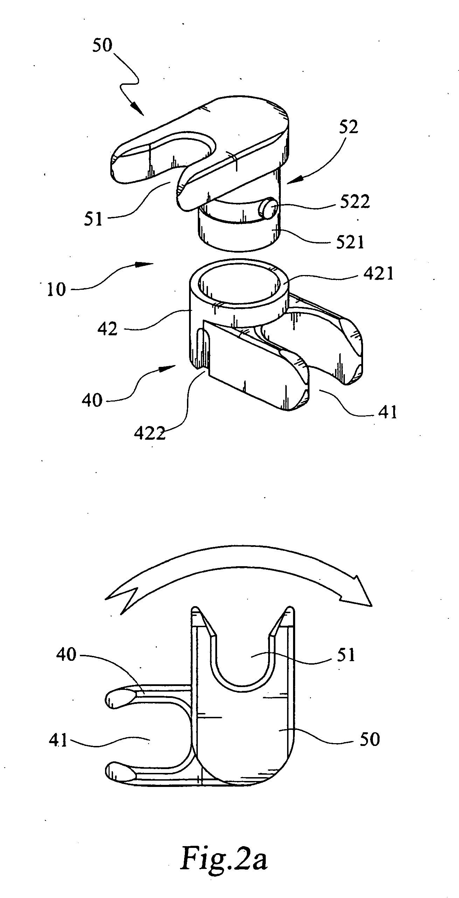

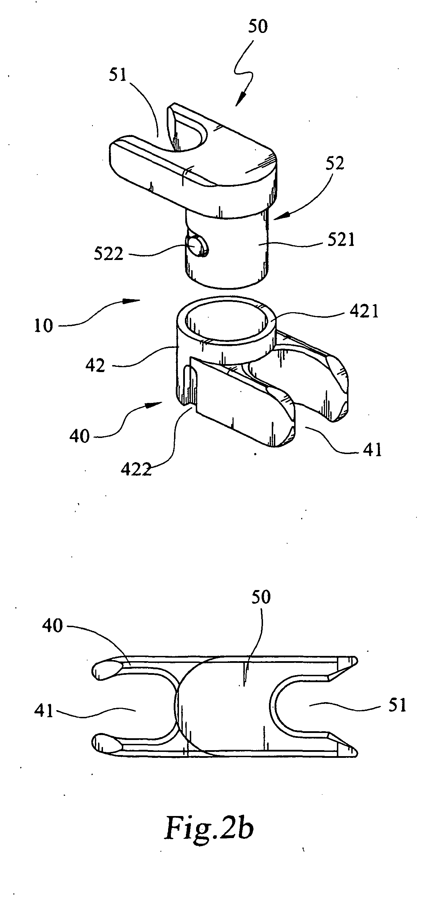

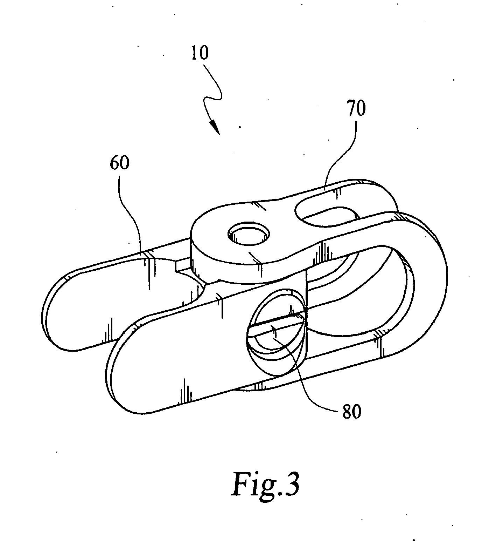

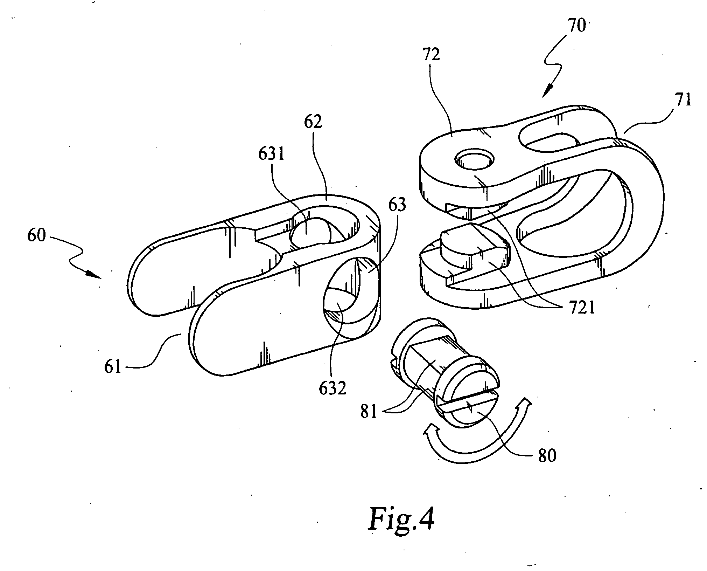

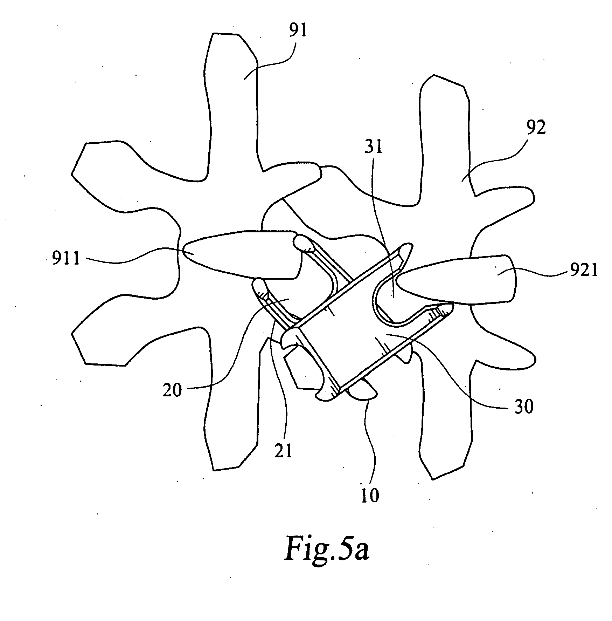

The structure and the technical means adopted by the present invention to achieve the above and other objects can be best understood by referring to the following detailed description of the preferred embodiments and the accompanying drawings, wherein: Fig. 1 is an exploded view that shows an implant for supporting spinous processes. Figs. 2a and 2b are schematic views that show another implant for supporting spinous processes, not part of the invention, but important for its understanding, while being assembled and rotated. Fig. 3 is a schematic view that shows an implant for supporting spinous processes according to the invention. Fig. 4 is an exploded view of the implant for supporting spinous processes shown in Fig. 3. Figs. 5a to 5c are schematic views that show the actual application of the implant to spinous processes.

### DETAILED DESCRIPTION OF PREFERRED EMBODIMENTS

The invention can be better understood by referring to the preferred embodiments and the accompanying drawings as described in the following paragraphs:
Fig. 1 shows an implant 10 for supporting spinous processes, which comprises a left prop 20 and a right prop 30. The left prop 20 is elongated and has a receiving end 21 at one end and a connecting end 22 at the other end. The receiving end 21 is U-shaped and may be applied directly to a spinous process, so as to fit and support the spinous process being treated. The connecting end 22 has another receiving end 23 extendingly formed at the end that faces away from the receiving end 21, and the connecting end 22 may be correspondingly fitted to a connecting end 32 of the right prop 30.

The right prop 30 is I-shaped and elongated; having a receiving end 31 at one end and the connecting end 32 at the other end. The receiving end 31 has an upper piece 311 and a lower piece 312; both have a U-shaped opening at the frontal end, which may be applied directly to another spinous process, so as to fit and support the another spinous process being treated. The connecting end 32 connects the upper piece 311 and the lower piece 312 together, and another receiving end 33 is extendingly formed at the end of the upper piece 311 that is at the junction with the connecting end 32 and faces away from the receiving end 31. The connecting end 32 may be correspondingly fitted to the connecting end 22 of the left prop 20.

Figs. 2a and 2b disclose another implant 10 for supporting spinous processes that comprises a left prop 40 and a right prop 50. The left prop 40 has a receiving end 41 and a connecting end 42, while the right prop 50 also has a receiving end 51 and a connecting end 52. The connecting end 52 of the right prop 50 further comprises an axis 521 having a fastening clip 522, whereas the connecting end 42 of the left prop 40 further includes an axial trough 421 having a fastening groove 422 formed on the inner surface thereof.

The left prop 40 and the right prop 50 are firstly allowed to connect to each other and form an angle by connecting the connecting end 42 and the connecting end 52 together, and the axis 521 is rotatably fitted into the axial trough 421. The fastening clip 522 is retracted by the pressing from the wall of the axial trough 421, and when the props 40 and 50 are rotated to particular angles in relation to each other, the fastening clip 522 springs out and clips into the fastening groove 422, thereby preventing the props 40 and 50 from rotating further, so that the props are kept linear as a whole and allowed to support the spinous processes being treated.

Fig. 3 shows an implant 10 for supporting spinous processes according to the invention, comprising not only a left prop 60, a right prop 70, and also a fixing member 80.

As shown in Fig. 4, the left prop 60 is also elongated and has a receiving end 61 at one end while a connecting end 62 at the other end. The receiving end 61 is U-shaped and may be applied directly to a spinous process, so as to fit and support the spinous process being treated. The connecting end 62 has a cross-shaped opening 63, which includes a vertical opening 631 and a horizontal opening 632, and the connecting end 62 may be correspondingly fitted to a connecting end 72 of the right prop 70.

The right prop 70 is elongated and has a receiving end 71 at one end and a connecting end 72 at the other end. The receiving end 71 is a U-shaped opening, which may be applied directly to another spinous process, so as to fit and support the another spinous process being treated. The connecting end 72 further includes a pair of upper and lower hooks 721, which may be rotatably fitted into the vertical opening 631 of the cross-shaped opening 63.

The fixing member 80 is a cylindrical piece with a flat surface 81 formed at two opposing sides thereof respectively, and the fixing member 80 may be fitted into the horizontal opening 632 of the cross-shaped opening 63.

When the fixing member 80 is fitted into the horizontal opening 632, and the two props 60 and 70 are already connected; when the fixing member 80 is rotated and allowed to have flat surfaces 81 thereof to become perpendicular to the surfaces of the upper and lower hooks 721 of the right prop 70, the props 60 and 70 may be rotated to left or right freely in relation to each other and allowed to form an angle, because the distance between the upper and lower hooks 721 is enlarged by two opposite sides that are separated by the flat surfaces 81 of the fixing member 80 and the upper and lower hooks are rotatably received in the vertical opening 631 of the left prop 60.

In contrast, when the props 60 and 70 are rotated to become linear in relation to each other, and when the fixing member 80 is so rotated that the flat surfaces 81 thereof become intimately adjacent to the surfaces of the upper and lower hooks 721 of the right prop 70, the distance between the upper and lower hooks 721 is reduced, and the upper and lower hooks 721 are fixedly engaged with the vertical opening 631 of the left prop 60 due to the shapes thereof, so that the props are kept linear as a whole and allowed to support the spinous processes being treated.

As shown in Fig. 5a, when the implant 10 is applied to support spinous processes, the receiving end 21 of the left prop 20 is allowed to hold a spinous process 911 of a upper vertebra 91, and the receiving end 31 of the right prop 30 is allowed to hold a spinous process 921 of a lower vertebra 92. Subsequently, the two props 20 and 30 are rotated in opposite directions to increase the angle between them, so that the props become linear as a whole.

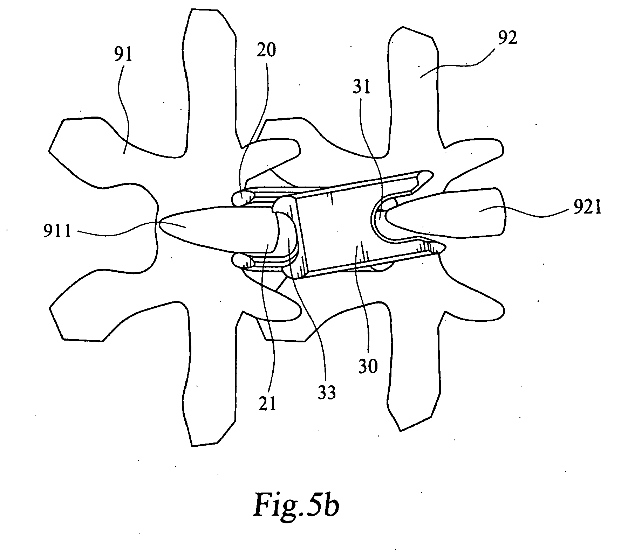

Referring to Fig. 5b, because the angle between props 20 and 30 have been increased to make the props 20 and 30 align linearly, the intervertebral space between the vertebrae 91 and 92 is consequently enlarged, and then the other receiving end 33 of the right prop 30 is aligned with the spinous process 911 of the upper vertebra 91.

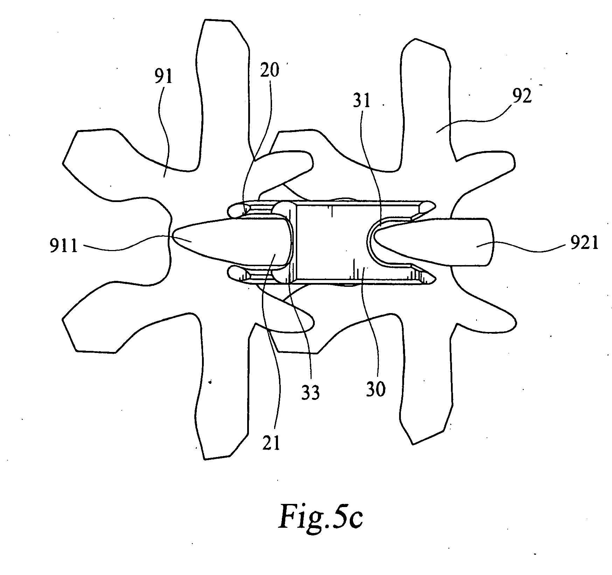

Now referring to Fig. 5c, once the implant 10 has been rotated to the desired position, the two props 20 and 30 are allowed to become linear as a result. This is followed by allowing the other receiving end 33 of the right prop 30 to hold the spinous process 911 of the upper vertebra 91, thereby completing the process of enlarging and supporting the intervertebral space between the vertebrae 91 and 92, and thus treating the problem of compressed spinous processes.

## Claims

1. An implant (10) for supporting spinous processes (911, 922), comprising:
a first prop (60) and a second prop (70), each having a first receiving end (61, 71) and a connecting end (62, 72), wherein said two props (60, 70) can be joined together at the connecting ends (62, 72) and rotated relatively in order to form an angle, while each of the first receiving ends (61, 71) is adapted to contact a spinous process (911, 922), and the joined props (60, 70) are able to be rotated in relation to each other, allowing the two props (60, 70) to become linear as a whole, so that an intervertebral space between two adjacent vertebrae (91, 92) with their spinous processes (911, 922) contacted by the receiving ends (61, 71) is enlarged and supported, **characterized in that**
the implant (10) further comprises a fixing member (80), wherein the connecting end (72) of the second prop (70) comprises an upper and a lower hook (721) and the connecting end (62) of the first prop (60) comprises a cross-shaped opening (63) having a horizontal opening (632) and a vertical opening (631), the fixing member (80) being rotatably received in the horizontal opening (632) and the upper and lower hooks (721) being rotatably received in the vertical opening (631), wherein the two props (60, 70) are able to be rotated to form an angle in relation to each other or to become linear as a whole when the fixing member (80) is rotated to a first position, and the upper and lower hooks (721) of the second prop (70) are fixedly received in the vertical opening (631) of the first prop (60) such that the two props (60, 70) are kept linear as a whole when the fixing member (80) is rotated to a second position.

2. The implant according to claim 1, wherein the fixing member (80) is a cylindrical piece with a flat surface (81) formed at two opposing sides thereof, the two flat surfaces (81) of the fixing member (80) separately contacting the upper and lower hooks (721) when the fixing member (80) is rotated to the second position, the cylindrical piece having cylindrical surfaces separated by the two flat surfaces (81) of the fixing member (80), said cylindrical surfaces contacting the upper and lower hooks (721) when the fixing member (80) is rotated to
the first position.

## Patentansprüche

1. Implantat (10) zum Stützen von Dornfortsätzen (911, 922), umfassend:
eine erste Stütze (60) und eine zweite Stütze (70), wobei jede ein erstes aufnehmendes Ende (61, 71) und ein verbindendes Ende (62, 72) aufweist, wobei die zwei Stützen (60, 70) miteinander an den verbindenden Enden (62, 72) verbunden und relativ rotiert werden können, um einen Winkel zu bilden, während jedes der ersten aufnehmenden Enden (61, 71) geeignet ist, um einen Dornfortsatz (911, 922) zu kontaktieren und die verbundenen Stützen (61, 70) sind in der Lage, relativ zueinander rotiert zu werden, wobei den zwei Stützen (60, 70) ermöglicht wird, im Gesamten geradlinig zu werden, so dass ein Zwischenwirbelraum zwischen zwei benachbarten Wirbelkörpern (91, 92) mit ihren Dornfortsätzen (911, 922), die von den zwei aufnehmenden Enden (61, 71) kontaktiert werden, vergrößert und gestützt wird, **dadurch gekennzeichnet, dass**
das Implantat (10) weiterhin ein Befestigungsbauteil (80) umfasst, wobei das verbindende Ende (72) der zweiten Stütze (70) einen oberen und einen unteren Haken (721) umfasst und das verbindende Ende (62) der ersten Stütze (60) eine kreuzförmige Öffnung (63) umfasst, die eine horizontale Öffnung (632) und eine vertikale Öffnung (631) aufweist, wobei das Befestigungsbauteil (80) rotierbar in der horizontalen Öffnung (632) aufgenommen ist und der obere und untere Haken (721) rotierbar in der vertikalen Öffnung (631) aufgenommen sind, wobei die zwei Stützen (60, 70) in der Lage sind, rotiert zu werden, um einen Winkel im Verhältnis zueinander zu bilden oder im Gesamten geradlinig zu werden, wenn das Befestigungsbauteil (80) zu einer ersten Position rotiert wird, und wobei der obere und untere Haken (721) der zweiten Stütze (70) fest in der vertikalen Öffnung (631) der ersten Stütze (60) aufgenommen sind, so dass die zwei Stützen (60, 70) im Gesamten geradlinig gehalten werden, wenn das Befestigungsbauteil (80) zu einer zweiten Position rotiert wird.

2. Implantat nach Anspruch 1,
**dadurch gekennzeichnet, dass**
das Befestigungsbauteil (80) ein zylindrisches Stück mit einer flachen Oberfläche (81) ist, die an zwei gegenüberliegenden Seiten davon ausgebildet ist, ist, wobei die zwei flachen Oberflächen (81) des Befestigungsbauteils (80) separat den oberen und unteren Haken (721) kontaktieren, wenn das Befestigungsbauteil (80) zu der zweiten Position rotiert wird, wobei das zylindrische Stück zylindrische Oberflächen aufweist, die durch die zwei flachen Oberflächen (81) des Befestigungsbauteils (80) getrennt sind, und wobei die zylindrischen Oberflächen den oberen und unteren Haken (721) kontaktieren, wenn das Befestigungsbauteil (80) zu der ersten Position rotiert wird.

## Revendications

1. Implant (10) pour le soutien des apophyses épineuses (911, 922), comprenant :
un premier étai (60) et un second étai (70), ayant chacun une première extrémité réceptrice (61, 71) et une extrémité de jonction (62, 72), dans lequel lesdits deux étais (60, 70) peuvent être reliés au niveau des extrémités de jonction (62, 72) et pivotés l'un par rapport à l'autre de façon à former un angle, tandis que chacune des premières extrémités réceptrices (61, 71) est conçue pour être en contact avec une apophyse épineuse (911, 922) et dans lequel les étais (60, 70) joints peuvent être tournés l'un par rapport à l'autre, permettant d'aligner les deux étais (60, 70) comme un tout, de façon à pouvoir élargir et soutenir l'espace intervertébral entre deux vertèbres (91, 92) adjacentes avec leurs apophyses épineuses (911, 922) au contact des extrémités réceptrices (61, 71), **caractérisé en ce que** :
l'implant (10) comprend en outre un élément de fixation (80) dans lequel l'extrémité de jonction (72) du second étai (70) comprend un crochet supérieur et un crochet inférieur (721) et dans lequel l'extrémité de jonction (62) du premier étai (60) comprend une ouverture (63) de forme transversale ayant une ouverture horizontale (632) et une ouverture verticale (631), l'élément de fixation (80) étant reçu de façon pivotante dans l'ouverture horizontale (632) et les crochets supérieur et inférieur (721) étant reçus de façon pivotante dans l'ouverture verticale (631), les deux étais (60, 70) pouvant pivoter pour former un angle l'un par rapport à l'autre ou se placer en ligne comme un tout lorsque l'élément de fixation (80) est pivoté dans une première position et les crochets supérieur et inférieur (721) du second étai (70) étant reçus fixement dans l'ouverture verticale (631) du premier étai (60) de façon à ce que les deux étais (60, 70) soient maintenus en ligne comme un tout lorsque l'élément de fixation (80) est pivoté dans une seconde position.

2. Implant selon la revendication 1, dans lequel l'élément de fixation (80) est une pièce cylindrique avec une surface plate (81) formée au niveau des deux côtés opposés de celle-ci, les deux surfaces plates (81) de l'élément de fixation (80) étant séparément en contact avec les crochets supérieur et inférieur (721) lorsque l'élément de fixation (80) est pivoté dans la seconde position, la pièce cylindrique ayant des surfaces cylindriques séparées par les deux surfaces plates (81) de l'élément de fixation (80), lesdites surfaces cylindriques étant en contact avec les crochets supérieur et inférieur (721) lorsque l'élément de fixation (80) est pivoté dans la première position.
